(19) Europäisches Patentamt
European Patent Office
Office européen des brevets

(11) Publication number: 0 178 168
B1

# (12) EUROPEAN PATENT SPECIFICATION

(45) Date of publication of patent specification: 17.01.90

(51) Int. Cl.$^5$: C 07 C 57/07, C 07 C 51/50

(21) Application number: 85307233.8

(22) Date of filing: 09.10.85

## (54) Process for purification of methacrylic acid.

(30) Priority: 10.10.84 US 659568

(43) Date of publication of application:
16.04.86 Bulletin 86/16

(45) Publication of the grant of the patent:
17.01.90 Bulletin 90/03

(84) Designated Contracting States:
AT BE CH DE FR GB IT LI LU NL SE

(56) References cited:
DE-A-1 618 141
DE-A-2 149 670
DE-A-2 202 980
FR-A-2 084 869

(73) Proprietor: AMOCO CORPORATION
200 East Randolph Drive
Chicago Illinois 60601 (US)

(72) Inventor: Lillwitz, Lawrence D.
773 Cresent Boulevard
Glen Ellyn Illinois 60137 (US)

(74) Representative: Ritter, Stephen David et al
Mathys & Squire 10 Fleet Street
London EC4Y 1AY (GB)

EP 0 178 168 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

Field of the Invention

This invention relates generally to the recovery of an alpha-, beta-ethylenically unsaturated monocarboxylic acid by distillation and, more particularly, concerns the inhibition of the polymerization of an alpha-, beta-ethylenically unsaturated monocarboxylic acid during its recovery by distillation.

Discussion of the Prior Art

Esters of alpha-, beta-ethylenically unsaturated monocarboxylic acids are large volume chemicals prepared by esterification of the corresponding alpha-, beta-ethylenically unsaturated monocarboxylic acids. In order to obtain such an ester of the desired purity, it is important that the alpha-, beta-ethylenically unsaturated monocarboxylic acid starting material is sufficiently pure. However, in the separation and purification of an alpha-, beta-ethylenically unsaturated monocarboxylic acid under distillation conditions, it has been found that the acid tends to polymerize even when conventional inhibitors are present.

For example, recently interest has developed in the production of an ester of an alpha-, beta-ethylenically unsaturated monocarboxylic acid by a process involving the reaction of a saturated aliphatic monocarboxylic acid of one less carbon atom and formaldehyde to form the alpha-, beta-ethylenically unsaturated monocarboxylic acid. The reaction results in a mixture of the alpha-, beta-ethylenically unsaturated monocarboxylic acid and the saturated aliphatic monocarboxylic acid of one less carbon atom, as well as other by-products such as alpha-, beta-unsaturated ketones, for example, 2,5-dimethylcyclopent-2-en-1-one. After separation of the alpha-, beta-ethylenically unsaturated monocarboxylic acid from the saturated aliphatic monocarboxylic acid of one less carbon atom, the alpha-, beta-ethylenically unsaturated monocarboxylic acid is esterified to form the ester of an alpha-, beta-ethylenically unsaturated monocarboxylic acid.

Esterification of the alpha-, beta-ethylenically unsaturated monocarboxylic acid with an alcohol in the presence of the saturated aliphatic monocarboxylic acid of one less carbon atom leads to the formation of an unwanted ester of the saturated aliphatic monocarboxylic acid of one less carbon atom. Therefore, it is essential to separate the saturated aliphatic monocarboxylic acid of one less carbon atom from the alpha-, beta-ethylenically unsaturated monocarboxylic acid prior to contacting the alpha-, beta-ethylenically unsaturated monocarboxylic acid with the alcohol. However, the separation of the alpha-, beta-ethylenically unsaturated monocarboxylic acid from the saturated aliphatic monocarboxylic acid of one less carbon atom by conventional methods such as distillation is difficult. For example, if distilled, the alpha-, beta-ethylenically unsaturated monocarboxylic acid polymerizes readily upon the application of heat. The resulting solid polymer deposits form in the distillation column and in the interconnecting lines. These deposits interfere with the efficient operation of the plant and eventually build up to such an extent that the plant must be shut down for cleaning.

Although the addition of inhibitors such as phenothiazine, benzoquinone, hydroquinone, 4-tertbutylcatechol, and p-phenylenediamine does reduce the polymerization of an alpha-, beta-ethylenically unsaturated monocarboxylic acid under these conditions, such inhibitors require the presence of oxygen in the distillation column, which can reduce the efficiency of the separation being effected in the column and can create an explosive mixture in the overhead condenser of the column. In addition, if the distillation were performed under reduced pressure, it would be difficult to keep a sufficient level of oxygen in the liquid phase.

Nitroxide radicals disclosed by O. L. Lebedev and S. N. Kazarnovski, Tr. po Khim. i. Khim. Tekhnol 2,649-654 (1959) have been demonstrated by Bailey, U.S. Patent No. 3,747,988, to be suitable stabilizers against the polymerization of acrylonitrile under distillation conditions and in either the presence or absence of oxygen — see also DE—2149670.

DE—1618141 discloses the use of nitroxide radicals to stabilise acrylic acid but warns that the monomer "will not necessarily be stabilised indefinitely by the presence of the nitroxide, especially when the monomer is heated as in distillation". Consequently such nitroxide radicals have never been employed to stabilize an alpha-, beta-ethylenically unsaturated monocarboxylic acid against polymerization under distillation conditions.

Related materials that have been employed to stabilize styrene and methyl methacrylate against polymerization at a temperature of 60°C during purification, storage and transport are disclosed in U.S.S.R. Inventor's Certificate No. 1027150 and are represented by the following general formula:

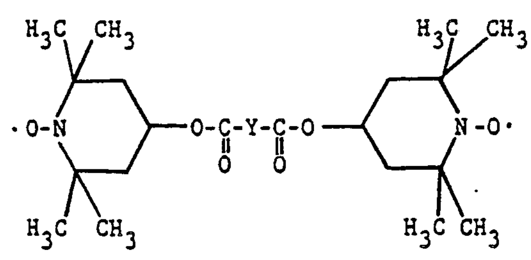

where Y is —NH—$(CH_2)_6$—NH— or

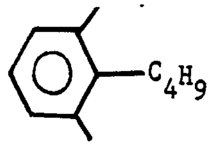

Stable free radicals can, in principle, both initiate and terminate radical polymerization reactions. With increasing temperature, their initiating action will become relatively more pronounced, because termination is a radical/radical reaction requiring zero or a low energy of activation, while the addition of a radical to a monomer usually requires energy of activation exceeding 5 Kcal./Mol. Thus, although stable nitroxide radicals are known, it is not obvious that the nitroxides of the present application would be particularly effective as polymerization inhibitors under distillation conditions for methyacrylic acid formed by the aldol-type condensation of proprionic acid and formaldehyde compound at relatively high temperatures (above 60°C) wherein the distillation feedstock comprises propionic acid and an alpha, beta-unsaturated ketone.

Thus according to the present invention, there is provided a process for the purification of methacrylic acid by distillation, which comprises adding to the distillation feedstock 2,2,6,6-tetramethyl-4-oxopiperidine-1-oxyl of the formula

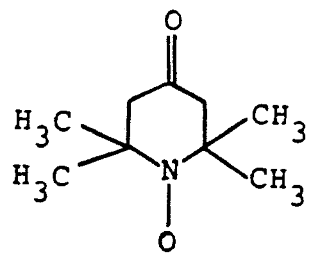

and conducting the distillation at a temperature in the range of from about 65°C to 175°C, wherein the distillation feedstock comprises propionic acid and an alpha-, beta-unsaturated ketone, and wherein the methyacrylic acid is prepared by the aldol-type condensation under vapor phase conditions at a temperature of about 280 to 350°C of (1) propionic acid and (2) formaldehyde compound in the presence of a solid catalyst.

The nitroxide radical

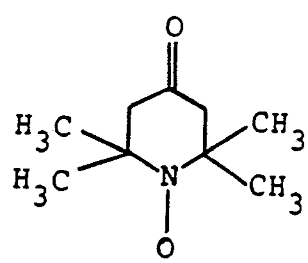

may be prepared by the methods disclosed by O. L. Lebedev and S. N. Kazarnovski, Tr. po Khim. i Khim. Tekhnol. 2, 649—656 (1959) and in U.S.S.R. Inventor's Certificate No. 1027150.

Suitably, the aforesaid nitroxide radical is employed in the method of this invention at a concentration in the range of from about 1 to about 10,000 preferably from about 50 to 5,000, parts per million parts of the alpha-, beta-ethylenically unsaturated monocarboxylic acid, and do not require the presence of oxygen.

The aforesaid nitroxide radical is suitable for use over a wide range of temperatures, in the range of from about 65°C to about 175°C, preferably from about 90°C to about 150°C, and more preferably from about 120°C to about 150°C. The method of the present invention is performed at an absolute pressure in the range of from about 10 to about 1200 millimeters of mercury.

Of the various alpha-, beta-ethylenically unsaturated compounds, it is generally recognized that methacrylic acid has one of the greatest tendencies to polymerize, and it is extremely difficult to handle at elevated temperatures. In this regard, it has been found that the presence of certain reaction by-products greatly increase the propensity of methacrylic acid to polymerize. Specifically, the alpha-, beta-unsaturated ketone by-products, such as ethylisopropenyl ketone and 2,5-dimethylcyclopent-2-en-1-one have been shown to greatly increase the degree of methacrylic acid polymerization.

For example, methacrylic acid alone polymerizes after 80, 19 and 8 minutes of exposure to temperatures of 100°C, 120°C and 140°C, respectively; while methacrylic acid in its mixture with 2,5-dimethylcyclopent-2-en-1-one containing 1 weight percent of 2,5-dimethylcyclopent-2-en-1-one polymerizes after only 18, 5 and 2.5 minutes of exposure to temperatures of 100°C, 120°C and 140°C,

respectively. In these instances, the time of polymerization is defined as the first appearance of a cloudy solution; precipitation of solids generally followed the first appearance of a cloudy solution within about 30 seconds. Therefore, the benefits from the use of the aforesaid nitroxide radical in the method of the present invention is crucial when an alpha-, beta-unsaturated ketone such as 2,5-dimethylcyclopent-2-en-1-one is present in the distillation feedstock.

The method of the present invention is employed for the recovery of methacrylic acid prepared by the aldol-type condensation under vapor phase conditions at a temperature of about 280 to 350°C of (1) propionic acid carbon atom than the alpha-, beta-ethylenically unsaturated, and (2) a formaldehyde compound in the presence of a solid catalyst. While any suitable source of formaldehyde compound can be used, such as formalin, paraformaldehyde, methanolic formaldehyde, trioxane, etc., it is preferred to use substantially anhydrous formaldehyde, particularly cracked monomeric gaseous, substantially anhydrous formaldehyde.

A large number of catalysts exhibit activity in such an aldol-type condensation reaction. Specific catalyst materials that are useful in the process include synthetic alkali metal aluminosilicates, natural alkali metal aluminosilicates, synthetic alkaline earth metal aluminosilicates, natural alkaline earth metal aluminosilicates, alkali metal hydroxides on synthetic aluminosilicates, alkali metal hydroxides on natural aluminosilicates, alkaline earth metal hydroxides on synthetic aluminosilicates, alkali metal hydroxides on silica gel, alkaline earth metal hydroxides on silica gel, sodium silicate on silica gel, potassium silicate on silica gel, molybdenum oxide on silica gel, silica gel, synthetic manganese aluminosilicate, natural manganese aluminosilicate, synthetic cobalt aluminosilicate, natural cobalt aluminosilicate, synthetic zinc aluminosilicate, and natural zinc aluminosilicate.

It is preferred to employ a catalyst comprising at least one cation of a Group I or Group II metal and a silica support. The alkali metal and/or alkaline earth metal cations of the catalyst can be used in a concentration of 0.01 to 0.20 equivalent, preferably 0.010 to 0.10 equivalent, of the cation per 100 grams of silica support, on a dry solids basis. Catalyst compositions found to be especially useful in the reaction are the subject of Hagen et al., United States patent application Serial No. 624,040, and Kaduk et al., United States patent application Serial No. 624,041, both filed June 25, 1984, both of which are specifically incorporated herein by reference in their entirety.

Suitable sources of alkali metal and alkaline earth metal cations include sodium hydroxide, potassium hydroxide, cesium hydroxide, lithium hydroxide, rubidium hydroxide, strontium hydroxide, magnesium hydroxide, lithium phosphate, trisodium phosphate, cesium phosphate, sodium borate, barium hydroxide, sodium carbonate, cesium fluoride, cesium nitrate, etc. Of these, the alkali metal cations are preferred.

While any commercially available colloidal silica can be used, it is preferred to use commercially available colloidal silicas having an average particle diameter of 40 to 1000 Angstrom units, particularly those having a particle diameter of about 50 to 250 Angstrom units, in order to produce silica-supported catalysts having a surface area of 20 to 275 $m^2/g$, a pore volume of 0.1 to 0.8 cc/g and an average pore diameter of about 75 to 200 Angstrom units, which are the subject of copending patent application Serial No. 624,040. As pointed out in patent application Serial No. 624,040, silica catalysts comprising at least one cation of a Group I or Group II metal and a silica support, said support having a surface area of 20 to 275 $m^2/g$, a pore volume of 0.1 to 0.8 cc/g and an average pore diameter of 75 to 200 Angstrom units have relatively high activity (that is, percent conversion and selectivity) and relatively long life. Pore volume, surface area and average pore diameter are interdependent variables. Other things being equal, holding one variable constant, as the surface area increases, pore volume increases; as the surface area increases, average pore diameter decreases; and as the pore volume increases, average pore diameter increases. It is critical in patent application Serial No. 624,040 that the catalyst satisfy each of the pore volume, surface area and average pore diameter requirements.

The silica can be treated either prior to drying or after calcination with cations. Catalysts prepared by the addition of the cations to the colloidal silica before or during gelation can be viewed as coformed catalysts which are the subject of copending patent application Serial No. 624,041. As pointed out in patent application Serial No. 624,041, coformed catalysts are advantageous in the sense that they are substantially more water tolerant than catalysts prepared by the treatment of the calcined silica with an aqueous solution of the cation and they are easier to decoke since, other things being equal, they do not exotherm as much as catalysts prepared by impregnation. This is apparently due to the more uniform distribution of the cations in the support.

The silica supports are preferably prepared by forming an aqueous composition comprising about 10 to 60 percent by weight colloidal silica on a dry basis and alkali metal and/or alkaline earth metal cation. The colloidal silica is gelled by adjusting the pH to a range of about 3 to 10, preferably about 6 to about 9, preferably with alkali metal or alkaline earth metal cations. Salts such as $NH_4NO_3$ can be used to accelerate gelation. While silica hydrogels can be aged for two weeks or more, aging seems to have no effect on the properties of the catalyst and accordingly, aging is not necessary. The composition is then dried by any suitable means, such as in a microwave oven, to constant weight and apparent dryness, for example, about 4 to 5 percent moisture on a dry solids basis. Apparently, only the water of hydration is retained by the silica gel after drying to constant weight. The silica gel is then calcined at about 300°C to 800°C, preferably about 300°C to 600°C.

In this preferred embodiment, the synthesis reactor feedstock should be composed of the propionic

acid, formaldehyde, and some water. The mole ratio of the propionic acid to formaldehyde should be maintained within the range of from about 25:1 to about 1:25; with a preferred range of from about 2:1 to about 1:2. The preferred concentration of water in the reactor (including water formed during the reaction) is at least 3 weight percent water in the reactor contents.

The synthesis reaction takes place over a wide temperature range of from about 280°C to about 500°C. Desirable and advantageous results are obtained by operating with temperatures in the range of about 280°C to about 350°C. The process is normally run at atmospheric pressure, although higher or lower pressures can be used.

The space velocity of the vaporized feed mixture over the catalyst may be varied over wide limits. Space velocity figures in this specification are based on the total number of moles of materials entering the catalyst zone. Total moles are multiplied by the volume of a mole of an ideal gas at 0°C and one atmosphere (22.4 liters/mole), to obtain the total volume. A space velocity in the range from about 100 liters per hour per liter of catalyst to about 1000 liters per hour per liter of catalyst is preferred.

The synthesis reactor effluent stream contains water of reaction, one mole of water for each mole of the methacrylic acid produced. An element of the preferred embodiment of the process of the invention is distillation of the reactor effluent stream under process conditions wherein 60—90 weight percent of the unreacted formaldehyde and 25—70 weight percent of the unreacted saturated monocarboxylic acid entering the distillation column are removed by a side-draw from the central part comprising from 10% to 90% of the theoretical trays of the distillation column for recycle to the synthesis reactor. The distillation column overhead consists of water, a small amount of formaldehyde and a trace of the propionic acid. The distillation column bottoms contain the methacrylic acid, the propionic acid and the heavy by-products of the synthesis reaction. The distillation column temperatures depend on the pressure employed and preferred distillation column conditions are:

| Column Temperatures | |
|---|---|
| Overhead °C | 71°—79° |
| Side-Draw, °C | 99°—121° |
| Bottoms, °C | 141°—157° |
| Column Pressure, Atm. | 1 |

The primary purpose of the reactor effluent distillation tower is to remove water from the synthesis reactor effluent. The overhead from this tower, consisting of formaldehyde, water and a small amount of the propionic acid, is sent to a formaldehyde recovery and dehydration section.

The bottoms of this tower consisting of the propionic acid reactant and unsaturated carboxylic acid product and heavy by-products are passed to a second distillation column operated under vacuum to separate the propionic acid as overhead from the methacrylic acid in the bottoms.

The present invention will be more clearly understood from the following specific examples.

## Examples 1—9

In each of Examples 1—9 an approximately 30-gram mixture containing propionic acid and methacrylic acid was introduced into a 3-necked 100-milliliter flask. 2,5-dimethylcyclopent-2-en-1-one (A) and the inhibitor 2,2,6,6-tetramethylpiperidine-1-oxyl (B) were also introduced. The lower half of the flask was inserted into a stirred oil bath which was maintained at the desired test temperature. The flask was equipped with a thermometer, reflux condenser and a sparge tube through which nitrogen was sparged into the solution. After the flask was immersed in the oil bath, the contents of the flask reached the test temperature in 5—7 minutes. The time when polymerization occurred was determined by withdrawing small aliquots of the mixture from the flask at frequent intervals and diluting each aliquot with an equal volume of dodecane to precipitate a polymer that formed. The time difference between the time when the solution in the flask reached the test temperature and the time when the slightest haze appeared in a diluted aliquot was defined as the time to polymerization. Precipitation of solids generally occurred in 30—60 seconds after the haze appeared.

The weight ratio of propionic acid-to-methacrylic acid in the solution in the flask was 60:40 in each of Examples 1—4 and 7—9, 63:37 in Example 5, and 61:39 in Example 6. The concentration of A in the solution in the flask was 1 weight percent in Examples 1—4 and 7—9 and 0.6 weight percent in Examples 5—6. The test temperature, weight percent of B in the solution in the flask, the weight percent of B in the solution in the flask divided by the weight percent of A in the solution in the flask, the number of parts of B per million parts by weight of methacrylic acid (MA) in the solution in the flask, and the time to polymerization for each of Examples 1—9 are presented in Table 1.

TABLE 1

| Example | Temp. (°C) | Conc. of B(Wt.%) | Conc. of B, based on A(Wt.%) | Conc. of B, based on MA(ppm) | Time (min) |
|---|---|---|---|---|---|
| 1 | 144 | 0.1 | 10 | 2549 | 23 |
| 2 | 143 | 0.05 | 5 | 1242 | 11 |
| 3 | 143 | 0.07 | 7 | 1783 | 18 |
| 4 | 143 | 0.1 | 10 | 2523 | 26 |
| 5 | 143 | 0.04 | 7 | 1145 | 16 |
| 6 | 138 | 0.06 | 10 | 1525 | 40 |
| 7 | 138 | 0.05 | 5 | 1325 | 20 |
| 8 | 138 | 0.1 | 10 | 2583 | 45 |
| 9 | 132 | 0.1 | 10 | 2550 | 65 |

Examples 10—20

In each of Examples 10—20, the procedure of Examples 1—9 was repeated, except that methacrylic acid alone, instead of a mixture of methacrylic acid and propionic acid, was introduced with A and B into the flask. The concentration of A in the solution in the flask was 1 weight percent in Examples 10—12, 3 weight percent in Examples 13—15 and 17—18, and 1.5 weight percent in Examples 16, 19 and 20. The test temperature, weight percent of B in the solution in the flask, the weight percent of B in the solution in the flask divided by the weight percent of A in the solution in the flask, the number of parts of B per million parts by weight of methacrylic acid (MA) in the solution in the flask, and the time to polymerization for each of Examples 10—20 are presented in Table 2.

TABLE 2

| Example | Temp. (°C) | Conc. of B(Wt.%) | Conc. of B, based on A(Wt.%) | Conc. of B, based on MA(ppm) | Time (min) |
|---|---|---|---|---|---|
| 10 | 132 | 0.1 | 10 | 1026 | 28 |
| 11 | 121 | 0.1 | 10 | 1000 | 105 |
| 12 | 116 | 0.1 | 10 | 1000 | 270 |
| 13 | 116 | 0.01 | 0.33 | 100 | <1 |
| 14 | 116 | 0.1 | 3.3 | 1000 | 90 |
| 15 | 116 | 0.05 | 1.7 | 500 | 10 |
| 16 | 116 | 0.075 | 5.0 | 760 | 150 |
| 17 | 99 | 0.03 | 1.0 | 306 | 105 |
| 18 | 99 | 0.02 | 0.7 | 200 | 45 |
| 19 | 99 | 0.03 | 2.0 | 315 | 183 |
| 20 | 121 | 0.1 | 7.0 | 1066 | 105 |

Examples 21—25

In each of Examples 21—25, the procedure of Examples 10—20 was repeated, except that in Examples 22, 23 and 25, 2,2,6,6-tetramethyl-4-oxopiperidine-1-oxyl was employed as the inhibitor instead of 2,2,6,6-tetramethylpiperidine-1-oxyl. The test temperature was 116°C in each of Examples 21—25. The concentration of A in the solution in the flask was 3 weight percent in Examples 21—23 and 1.5 weight percent in Examples 24—25. The concentration of the inhibitor in the solution in the flask, the concentration of the inhibitor in the solution in the flask divided by the weight percent of A in the solution in the flask, the number of parts by weight of the inhibitor per million parts of methacrylic acid (MA) in the solution in the flask, and the time to polymerization for each of Examples 21—25 are presented in Table 3.

TABLE 3

| Example | Conc. of Inhibitor (Wt.%) | Conc. of Inhibitor, based on A(Wt.%) | Conc. of B, based on MA(ppm) | Time (min.) |
|---|---|---|---|---|
| 21 | 0.1 | 3.3 | 1000 | 90 |
| 22 | 0.1 | 3.7 | 1144 | 60 |
| 23 | 0.1 | 3.7 | 1147 | 60 |
| 24 | 0.075 | 5.0 | 760 | 150 |
| 25 | 0.075 | 5.5 | 831 | 90 |

Examples 26—27

In each of Examples 26 and 27, a feed comprising propionic acid and formaldehyde in a molar ratio of 0.95 moles of propionic acid per mole of formaldehyde was fed continuously to a vapor phase reactor containing a bed of a catalyst comprising 5 weight percent of cesium hydroxide (calculated as elemental cesium) on silica having a surface area of 150 $m^2/g$ and maintained at a temperature of 324°C and a pressure of 4 psig. The propionic acid and formaldehyde reacted to form methacrylic acid, and the reactor effluent was fed to a first distillation column which was operated at atmospheric pressure and an overhead temperature of 76°C, a side-draw temperature of 115°C and a bottoms temperature of 147°C and contained 40 inches of packing material disposed above 20 oldershaw trays. The mixture was introduced at the level of the 18th tray from the bottom. The polymerization inhibitor was dissolved in propionic acid and introduced into this column at the level of the 20th tray from the bottom. Methacrylic acid, propionic acid and heavy reaction by-products were recovered as a bottoms product, and passed to a second distillation column which was operated at a pressure of 100 millimeters of mercury (measured at the top of the column), an overhead temperature of 87°C and a bottoms temperature of 116°C and contained 35 oldershaw trays. The polymerization inhibitor was dissolved in propionic acid and introduced into this column near its top. Methacrylic acid and heavy reaction by-products were recovered as a bottoms product.

The system was operated at steady state, and the following data were collected over a 2-hour period during steady state operation. In each of Examples 26 and 27, approximately 3,170 grams of propionic acid and 1,373 grams of formaldehyde were fed to the vapor phase reactor during the 2-hour period. In Example 26, the first and second distillation columns were operated at reflux ratios of 1.9 and 3.6, respectively. In Example 27, the first and second distillation columns were operated at reflux ratios of 2.2 and 2.9, respectively. 3.22 grams and 1.31 grams of 2,2,6,6-tetramethyl-4-oxopiperidine-1-oxyl as the inhibitor were introduced into the first and second distillation columns, respectively, in Example 26 during the 2-hour period. 2.89 grams and 1.19 grams of 2,2,6,6-tetramethylpiperidine-1-oxyl as the inhibitor were introduced into the first and second distillation columns, respectively, in Example 27 during the 2-hour period. In Examples 26 and 27, the bottoms from the second distillation column weighed 1,189 grams and 1,122 grams, respectively, of which approximately 85 weight percent was methacrylic acid. 0.68 weight percent and 0.63 weight percent of the bottoms from the second distillation column in Examples 26 and 27, respectively, were precipitated as polymeric solids upon dilution with an equal volume of dodecane.

The results of Examples 1—20 illustrate that the polymerization time is a function of the distillation temperature. At a particular distillation temperature, the polymerization time varies inversely with the concentration of the polymerization inhibitor divided by the concentration of the polymerization accelerator (A). Obviously the concentration of the polymerization inhibitor that should be employed to effect the desired inhibition depends on the identities of the alpha-, beta-ethylenically unsaturated monocarboxylic acid, of the polymerization accelerator (for example, 2,5-dimethylcyclopent-2-en-1-one)

present, and of the inhibitor employed, the amount of polymerization accelerator present, the elevated temperature, and the time for which the alpha-, beta-ethylenically unsaturated monocarboxylic acid is exposed to the elevated temperature.

Furthermore, by contrast to the steady state operations illustrated by Examples 26 and 27, when a nitroxide free radical polymerization inhibitor of the type employed in the method of this invention was not employed, polymerization and deposition of solid polymers occurred so quickly that the system could not be operated long enough to establish steady state conditions.

From the above description, it is apparent that the objects of the present invention have been achieved. While only certain embodiments have been set forth, alternative embodiments and various modifications will be apparent from the above description to those skilled in the art. These and other alternatives are considered equivalents and within the spirit and scope of the present invention.

Having described the invention, what is claimed is:

**Claims**

1. A process for the purification of methacrylic acid by distillation, which comprises adding to the distillation feedstock 2,2,6,6-tetramethyl-4-oxopiperidine-1-oxyl of the formula

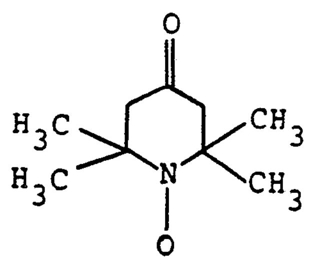

and conducting the distillation at a temperature in the range of from about 65°C to 175°C, wherein the distillation feedstock comprises propionic acid and an alpha-, beta-unsaturated ketone, and wherein the methacrylic acid is prepared by the aldol-type condensation under vapor phase conditions at a temperature of about 280 to 350°C of (1) propionic acid and (2) a formaldehyde compound in the presence of a solid catalyst.

2. A process according to Claim 1, wherein the distillation feedstock comprises from about 1 to about 10,000 parts of 2,2,6,6-tetramethyl-4-oxopiperidine per million parts of methacrylic acid.

**Patentansprüche**

1. Verfahren zur Reinigung von Methacrylsäure durch Destillation, dadurch gekennzeichnet, daß der Destillationssumpf mit 2,2,6,6-Tetramethyl-4-oxopiperidin-1-oxyl der Formel

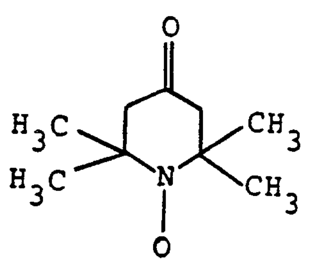

versetzt und die Destillation bei einer Temperatur im Bereich von etwa 65°C bis 175°C durchgeführt wird, wobei ein Destillationssumpf verwendet wird, der Propionsäure und ein alpha-, beta-ungesättigtes Keton enthält und die Methacrylsäure durch Aldol-Kondensation unter Dampfphasenbedingungen bei einer Temperatur von etwa 280°C bis 350°C von (1) Propionsäure und (2) einer Formaldehydverbindung in Gegenwart eines festen Katalysators hergestellt worden ist.

2. Verfahren nach Anspruch 1, dadurch gekennzeichnet, daß der Destillationssumpf etwa 1 bis etwa 10.000 Teile 2,2,6,6-Tetramethyl-4-oxopiperidin-1-oxyl pro Million Teile Methacrylsäure enthält.

**Revendications**

1. Procédé pour la purification de l'acide méthacrylique par distillation, qui comprend l'addition à la charge de distillation du 2,2,6,6-tétraméthyl-4-oxopipéridine-1-oxyle de formule

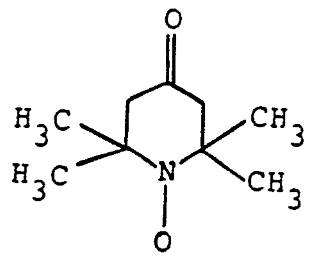

et la réalisation de la distillation à une température située dans la gamme d'environ 65°C à 175°C, dans lequel la charge de distillation comprend de l'acide propionique et une cétone alpha-, béta-insaturée, et dans lequel l'acide méthacrylique est préparé par la condensation de type aldol, dans des conditions de phase gazeuse à une température d'environ 280 à 350°C, (1) de l'acide propionique et (2) d'un composé du formaldéhyde en présence d'un catalyseur solide.

2. Procédé selon la revendication 1, dans lequel la charge de distillation comprend d'environ 1 à environ 10000 parties de 2,2,6,6-tétraméthyl-4-oxopipéridine par million de parties d'acide méthacrylique.